# EUROPEAN PATENT APPLICATION

(11) **EP 1 986 114 A1**
(43) Date of publication of application: **29.10.2008**
(21) Application number: 07105663.4
(22) Date of filing: 04.04.2007
(51) Int. Cl.: G06F 19/00

(54) **Adaptive disease modeling**

(71) Applicant: Koninklijke Philips Electronics N.V., 5621 BA Eindhoven (NL)
(72) Inventor: The designation of the inventor has not yet been filed
(74) Representative: Golla-Franz, Anke Lucia

(57) **Abstract**

The invention relates to a method and a computer program for the simulation of a physiological system, particularly in the state of a disease. According to this approach, the behavior of the system is at least partially expressed in a model with probability distributions according to the Bayesian theory. The output of the simulation is thus described by probability distributions, too, yielding a more realistic description than deterministic simulations of an average system. Moreover, the model can readily be updated with patient specific data and new scientific findings by expressing the confidence in the new information with probability distributions.

## Description

The invention relates to a method for the simulation of a physiological system, particularly in the state of a disease, as well as to a computer program enabling such a method and a record carrier comprising such a computer program.

Modem day diagnostics can draw from a large variety of patient data, including molecular (high throughput) data, (molecular) imaging and huge amounts of background knowledge. Because of the sheer size and complexity of this multi-modal avalanche of medical measurements and knowledge, human physicians are no longer able to form a complete picture in their head of everything that is known about (or relevant for) the patient that they are treating. A modem day hospital will therefore support the physician with digital patient records, easy access knowledge bases (on-line libraries and handbooks) and clinical decision support systems (CDSS).

A problem with many existing disease models and CDSS's is that the model, before it is "put to work" in the hospital, is optimized based on the current state of the art knowledge (at the moment of model creation) and on one or more clinical studies. The effect of the latter part of optimization is that the model performs well for (gives an accurate description of) the "average" patient, as defined through the clinical study that was used. The average patient does of course not exist in real life, and any new patient that enters the hospital will therefore deviate from the predefined model to some extent. This last issue has been addressed in the PCT/US2004/033130, where a group of multiple "virtual patients" is created, to reflect the biological patient to patient variability in a disease model. This method, however, is still only an approximate solution, since it is unlikely that such a set of virtual patients can reliably cover the full biological variance, and a discrepancy, albeit a smaller one, between the real and the closest simulated patient will remain. Furthermore, the question of which patient is closest is not easy to answer, since a difference with respect to one observed biological value may be more important than the difference with respect to another, less relevant value.

Based on this background it was an object of the present invention to provide means for an improved simulation of physiological systems, particularly of diseases, wherein these means should preferably allow a patient specific adaptation and/or and easy update with respect to new scientific insights.

This object is achieved by a method according to claim 1, by a computer program according to claim 4, and by a record carrier according to claim 9. Preferred embodiments are disclosed in the dependent claims.

The method according to the present invention serves for the simulation of a physiological system like a human or animal body, organ, or other subsystem. Preferably it is intended for the simulation of a physiological system in the state of a specific disease. According to the method the behavior of the physiological system is at least partially expressed in a mathematical model with probability distributions according to the Bayesian theory. The application of Bayesian theory to the description of complex systems is known to a person skilled in the art and has been described in literature (e.g. Weber, P. and Jouffe, L., "Complex system reliability modelling with Dynamic Object Oriented Bayesian Networks (DOOBN)", Reliability Engineering & System Safety, Volume 91, Issue 2, Pages 149-162 (2006); Smith, A., Jarvis, E. and Hartemink, A., "Evaluating functional network inference using simulations of complex biological systems", Bioinformatics, Volume 18, Suppl. 1, 2002, Pages S216-S224 (2002); Golightly, A., Wilkinson, D., "On Bayesian inference for stochastic kinetic models using diffusion approximations", Biometrics, Volume 61, Issue 3, Pages 781-788 (2004)).

Describing a physiological system and particularly a specific disease with the help of probability distributions has the advantage that the outputs of the simulation are characterized by probability distributions, too, thus providing a reasonable description of patient-to-patient variations and not being limited to the unrealistic assumption of an average patient.

The mathematical model may particularly comprise the description of molecular mechanisms like chemical reactions. Such models become more and more available due to the increasing biochemical knowledge.

According to a further development of the method, patient specific data and/or new information about the system behavior (obtained for example from scientific publications or clinical studies) are added to the model with probability distributions that reflect the confidence in these data or information. Thus a particular advantage of the Bayesian theory is exploited, namely the easy and intuitive possibility to extend and update a model.

The invention further relates to a computer program enabling the execution of a method of the kind described above.

In a further development, that computer program comprises a "visualization module" for visualizing data of the model, for example parameters or simulated quantities like molecule concentrations, blood pressure or the like. This provides an intuitive way for a user to learn about the results of the simulation and to observe the effects of changes in the model.

In another embodiment, the computer program comprises a "tuning module" for setting the relative confidence in different model components. Thus a user can particularly adjust the way in which an existing model is combined with new information, for example from scientific publications or clinical studies.

The computer program may further optionally comprise an "update module" for assisting the addition of patient-specific data and/or new information about the system behavior to the model. The model can then always be kept up-to-date with respect to new scientific findings and it can optimally be adapted to individual patients to which it shall be applied.

The aforementioned update module preferably comprises text mining algorithms for extracting new information from relevant texts, for example scientific publications. More information on such algorithms may be found in literature (e.g. Chen, H. and Sharp, B., "Content-rich biological network constructed by mining PubMed abstracts", BMC Bioinformatics, Volume 5:147 (2004); Krallinger, M., Erhardt, R. and Valencia, A., "Text-mining approaches in molecular biology and biomedicine", Drug Discovery Today, Volume 10, Issue 6, Pages 439-445(2005)).

Finally, the invention comprises a record carrier, for example a floppy disk, a hard disk, or a compact disc (CD), on which a computer program of the aforementioned kind is stored.

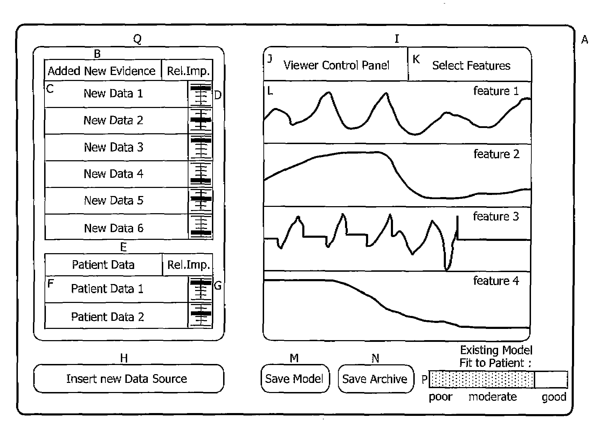

These and other aspects of the invention will be apparent from and elucidated with reference to the embodiment(s) described hereinafter. These embodiments will be described by way of example with the help of the accompanying single drawing which shows an example of the user interface for an adaptive model according to the present invention.

Disease models that describe the expected (molecular) characteristics of a patient who suffers from a specific disease can be used in clinical diagnosis, prognosis and monitoring. Current disease models are based on general knowledge about the disease (the current state of the art), augmented with the results of relevant clinical studies. Such models describe the "average patient", since one model must provide an overall good description of the test subjects in the clinical studies. Furthermore, these models will become more and more obsolete in time, since the state of the art at the moment that the model was created will be replaced by new research findings and new insights. This invention proposes a method and framework to adapt such models both to the specific patient under diagnosis, and to changes in the relevant state of the art.

The current invention aims to achieve this through the use of a Bayesian disease model that is able to automatically "learn" from new data (be it new knowledge in the field or new clinical studies) and to adapt to a new patient, thus producing a "patient specific disease model". The invention also includes a graphical user interface device that enables the medical scientist to control, monitor and influence the adaptation of the model to new data and to new patients.

Bayesian statistics, which has been widely used in the area of machine learning and information theory, provides a sound mathematical framework to build (or update) a model from the combination of already known data (a priori knowledge) and new evidence (e.g. patient specific clinical data, and new findings or clinical studies published in literature). A key element of the Bayesian approach to adaptive disease modeling is that both the current model and the new evidence are expressed in terms of probability distributions. This means that the current disease model will not only provide the most likely prediction for a new patient (corresponding to the "average" patient in deterministic modeling), but also a range of other possible predictions and their relative likelihoods. Such a distribution reflects both the biological variability and the extent to which the model is "confident" about its predictions.

Bayesian methods provide a way to update the model with new findings, and to personalize the model with respect to a new patient. However, all new evidence needs to be accompanied by a probability distribution or "confidence interval", indicating for example how certain it is that a new scientific finding is correct, or what the expected measurement inaccuracy (noise) is for a new patient measurement. With these two requirements satisfied, clear mathematical equations exist to express the likelihood of any prediction for a future patient based on the old model and the new data.

The specific implementation of the original probabilistic disease model depends very much on the (biomedical) contents of the model, but should be feasible for any scientist skilled in the art of mathematics and probability theory (and of course with a thorough understanding of the contents of the model). Note that this model can be developed before use in the hospital, and can draw on all experts available within the company that offers the modeling solution. The more difficult part lies in the expression of new scientific findings, clinical studies and patient specific clinical data in terms of probability distributions, which must be done after the disease model or CDSS software has been installed in the hospital. Not only may one not expect a physician to be a skilled mathematician or probability theorist as well, but even for such a hypothetical overall expert the expression of "confidence" in new findings or measurements is a highly subjective matter. Varying the confidence in newly acquired data (i.e. varying the variance terms in the corresponding probability distributions) can however be seen as varying the relative influence of the old model and of the new data on the new, adapted model, which is more intuitive. The remainder of this document will therefore describe a tool to support the physician in understanding the current model, adding new (patient specific) data and finding the right balance between the old, general model, new findings and patient specific clinical data. Possible clinical uses of such a tool are:
- Diagnosis: compare different models for different patient condition (e.g. healthy, disease variant 1 and disease variant 2). Have each of the models predict or simulate new clinical data for the patient (e.g. vital signs, certain protein concentrations in the blood, etc). The patient is diagnosed with the condition for which the predictions of the corresponding model coincide most closely with the actually measured patient data (or as inconclusive if none of the models match).
- Prognosis: insert the clinical data for a patient with a known condition into a dynamic (time dependent) model, and predict what is going to happen to the patient in the future.
- Monitoring: (follow-up example:) check the concentrations of certain relevant molecules in the blood stream of a post-radiotherapy patient, and see whether this clinical data corresponds to the modeled results of a successful therapy.

The aforementioned modeling approach is preferably realized with a computer program comprising a user interface that allows the medical expert to
- View the model before, after and during model update, e.g. by monitoring model output or viewing model parameters directly.
- Insert new data in the form of new findings, clinical study results or single patient data that are relevant to the model.
- Tune the relative importance of the new data added, as compared to the importance of (trust in) the existing model.

An example of the graphical user interface of such a program is schematically shown in the Figure. This interface comprises:
A workspace (A), either on a computer screen (virtual) or in the form of a (hardware) control panel.
A model output panel (I), where the current model (possibly including any alterations made by the user during the current working session) can be analyzed. This panel should include
   - A view screen (L) where various outputs of the model can be plotted or otherwise shown, and where further messages to support the user can appear.
   - A menu button (K) that allows the user to select features of the model (e.g. the concentration of a specific molecule over time) that will be shown on the view screen (L).
   - A further menu button (J) that accesses a viewer control panel, including such options as "zoom", "log transform", "play as movie" (for time series data), etc.
A button (H) that leads to a "Wizard" that guides the user through the process of inserting new data. The wizard should provide a clear format and upload option for new clinical or patient data, and easy options for the inclusion of new scientific findings. The latter could be provided through a series of questions (e.g. what molecules or other model aspects are involved, what is the type of interaction between the molecules and what is the corresponding reaction rate) or through a graphical interface (e.g. a visualization of the relevant pathways accompanied by point and click selection of parts of the model that are to be updated). An alternative option is to ask for a link to the article or study that describes the relevant new knowledge, and use intelligent text mining algorithms to synthesize a hypothesis for the corresponding model update, which can subsequently be edited by the user.
A panel (Q) that allows the user to define the desired balance between the old model and the added new data. This panel is divided into
   - A sub-panel (B) for the added sources of knowledge about the model, containing
      -- A number of text fields (C) that describe the added knowledge
      -- Corresponding sliders (D) that can be used to tune the relative importance/influence of each element.
      To facilitate further fine-tuning, the existing model (before adaptation) could be set as one of the panels inside B. The resulting changes (due to balance tuning) are reflected directly in the plots and figures on the view screen (L).
   - A sub-panel (E) to include the data of a specific new patient. This panel is similarly divided into panels F and G to describe and tune one or more sources of patient specific clinical data.
A menu option (M) that writes the adapted model to disk.
A button (N) that facilitates the retrieval of all previous versions of the model (one or several adaptation sessions back) from the archive.
A gauge (P) that indicates to the user how well a new patient is described by the current model. There may be situations where this fit is rather poor, either because the model itself is bad (e.g. since not enough relevant knowledge has been added to it or because the physician accidentally selected the wrong model) or because the patient is an outlier, i.e. does not compare well to "normal" patients with the same disease. A "poor" readout on this gauge may cause a physician to decide to rely on conventional diagnostics instead.

The invention can be used in a clinical setting, preferably in diagnostics/prognostics. There is however also a strong link to medical science, where such a tool can be used by a medical scientist without a background in mathematics or computer science. The invention is preferably embedded in a (computer) system that is linked to various (molecular) measurement and monitoring devices, but also to hospital information systems and local and on-line knowledge bases. The method provides a "living" disease model, that can be updated to the latest state of the art and local knowledge in a continuous fashion, a way to adapt (a copy of) the disease model toward a patient specific model and a graphical user interface to allow a medical expert to perform such model adaptations in a simple, intuitive and swift fashion.

Finally it is pointed out that in the present application the term "comprising" does not exclude other elements or steps, that "a" or "an" does not exclude a plurality, and that a single processor or other unit may fulfill the functions of several means. The invention resides in each and every novel characteristic feature and each and every combination of characteristic features. Moreover, reference signs in the claims shall not be construed as limiting their scope.

## Claims

1. A method for the simulation of a physiological system, particularly in the state of a disease, wherein the behavior of the system is at least partially expressed in a mathematical model with probability distributions according to the Bayesian theory.

2. The method according to claim 1,
**characterized in that** the mathematical model describes molecular mechanisms.

3. The model according to claim 1,
**characterized in that** patient specific data and/or new information about the system behavior are added to the model with probability distributions that reflect the confidence into these data or information.

4. A computer program for enabling carrying out a method according to claim 1.

5. The computer program according to claim 4,
**characterized in that** it comprises a visualization module for visualizing data of the model.

6. The computer program according to claim 4,
**characterized in that** it comprises a tuning module for setting the relative confidence of different model components.

7. The computer program according to claim 4,
**characterized in that** it comprises an update module for assisting the addition of patient specific data and/or new information about the system behavior to the model.

8. The computer program according to claim 7,
**characterized in that** the update module comprises text mining algorithms for extracting new information.

9. A record carrier on which a computer program according to claim 4 is stored.
